(19) Europäisches Patentamt
European Patent Office
Office européen des brevets

(11) Numéro de publication: **0 504 592 B1**

(12) **FASCICULE DE BREVET EUROPEEN**

(45) Date de publication de fascicule du brevet: **10.05.95**

(51) Int. Cl.$^{6}$: **C07C 35/21**, C07C 49/653, C07C 29/40, A61K 7/00

(21) Numéro de dépôt: **92102553.2**

(22) Date de dépôt: **15.02.92**

# (54) Alcool tertiaire à sous-structure campholénique et son utilisation à titre d'ingrédient parfumant.

(30) Priorité: **22.03.91 CH 890/91**

(43) Date de publication de la demande:
**23.09.92 Bulletin 92/39**

(45) Mention de la délivrance du brevet:
**10.05.95 Bulletin 95/19**

(84) Etats contractants désignés:
**CH DE FR GB LI NL**

(56) Documents cités:
**WO-A-80/00915**
**WO-A-81/00845**
**DE-B- 2 935 683**
**US-A- 4 174 287**
**US-A- 4 278 569**

**RECUEIL DES TRAVAUX CHIMIOUES DES PAYS-BAS. vol. 106, no. 1, Janvier 1987, DEN-HAAG NL pages 29 - 34; J.G. WITTEVEEN ET AL.: 'Structure-odour relationships of some new synthetic sandalwood aroma chemicals. Synthesis and olfactive properties in a series of bicyclo[4.4.0]decan-3-ols'**

(73) Titulaire: **FIRMENICH SA**
**1, route des Jeunes**
**CH-1211 Genève 8 (CH)**

(72) Inventeur: **Chapuis, Christian**
**5, Vy de Montorge**
**CH-1295 Mies (CH)**

(74) Mandataire: **Salvadori, Giuseppe, Dr.**
**c/o Firmenich S.A.**
**Case Postale 239**
**CH-1211 Genève 8 (CH)**

**EP 0 504 592 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a trait à des composés nouveaux possédant des propriétés odorantes utiles et, plus particulièrement, à un alcool tertiaire dérivé de l'aldéhyde campholénique et dont l'odeur est de type santalé.

L'ensemble des composés à odeur santalée constitue un domaine parfumistique dont l'art antérieur est riche en exemples. Il n'en reste pas moins vrai, cependant, qu'il s'agit aussi d'un domaine odorant où la recherche de nouveaux produits est toujours très active, ceci en raison du fait que l'essence naturelle du bois de santal est un ingrédient très apprécié, mais coûteux et disponible en des quantités limitées. Il s'ajoute à cela la difficulté que l'on retrouve à reproduire, même avec des mélanges de produits synthétiques connus, l'odeur de l'ingrédient naturel. On constate en effet que chaque composé de l'art antérieur, qui est à même d'apporter une note santalée aux compositions auxquelles il est incorporé, ne contribue à lui seul qu'à l'un ou l'autre des caractères particuliers à l'essence naturelle du bois de santal, et ceci dans une mesure plus ou moins grande. Aucun de ces composés ne présente une odeur identique à celle d'un autre et chacun d'eux peut trouver un emploi bien particulier en parfumerie, enrichissant ainsi la palette du parfumeur dans le domaine des notes santalées, sans pourtant la saturer.

D'autre part, en dépit des règles établies par différents chercheurs pour définir les relations entre la structure moléculaire et l'odeur de type bois de santal des composés connus, règles qui se voulaient utiles dans la prévision des qualités olfactives de nouveaux composés sur la base de leur structure moléculaire [voir, par exemple, E. J. Brunke et E. Klein, Essential Oils, Ed. B. D. Mookherjee et C. J. Mussinan, page 83 et suivantes, Allured Publishing Corp. (1981); R. E. Naipawer et al., ibid., page 105 et suivantes ; J. G. Witteveen et al., Recl. Trav. Chim. Pays-Bas 106, 29 (1987)], force est de constater que ces règles établissent des exigences structurelles qui, bien que paraissant essentielles, ne sont pas suffisantes pour permettre de prévoir qu'une structure moléculaire déterminée impliquera une odeur santalée certaine ou, qui plus est, d'une qualité, intensité et tenacité désirables [voir, par exemple, J. G. Witteveen, référence citée ou, encore, le brevet US 4,052,341].

Pour ces raisons, la recherche de nouveaux composés à odeur santalée reste toujours d'actualité et le nombre de brevets accordés jusqu'à ce jour dans ce domaine, brevets qui reconnaîssent non seulement la nouveauté mais aussi le caractère inventif de composés dont la structure est parfois presque identique à celle des composés déjà connus, est une mesure de l'importance attribuée à la découverte de nouveaux composés de ce type, dont les propriétés odorantes présentent des avantages certains par rapport à celles des composés décrits auparavant.

La présente invention apporte justement une nouvelle solution à ce problème. Elle concerne, plus particulièrement, un alcool tertiaire dérivé de l'aldéhyde campholénique, à savoir, le 1,2-diméthyl-4-(2',2',3'-triméthyl-3'-cyclopentén-1'-yl)-2-cyclohexén-1-ol.

Nous avons découvert que ce composé possède une note boisée puissante, avec un net côté santalé très propre qui se développe pour devenir plus puissant en fond. Il s'agit, en fait, d'une note très originale, qui se révèle être d'une excellente ténacité et qui est également plus puissante que celle de la plupart des composés de type boisé-santalé qui existent actuellement sur le marché.

Par exemple, lorsque comparé au (1'R,E)-3,3-diméthyl-5-(2',2',3'-triméthyl-3'-cyclopentén-1'-yl)-4-pentén-2-ol, un produit fort apprécié des parfumeurs et jugé supérieur à bien d'autres produits commerciaux à odeur santalée [voir, par exemple, EP 0 155 591], le composé de la présente invention se révèle être d'une utilisation plus facile en composition, notamment pour des applications en parfumerie fine. Ceci résulte du fait que, bien que possédant une note légèrement moins puissante que celle du composé connu cité, le composé de l'invention exhale une odeur de bois de santal plus propre et naturelle, et aussi moins animale, que celle du composé de l'art antérieur cité.

Cette supériorité olfactive du 1,2-diméthyl-4-(2',2',3'-triméthyl-3'-cyclopentén-1'-yl)-2-cyclohexén-1-ol apparaît comme surprenante et inattendue si l'on considère sa structure moléculaire. En effet, malgré le nombre élevé de composés santalés connus qui ont une sous-structure campholénique, il s'agit presque exclusivement d'alcools primaires ou secondaires dérivés de l'aldéhyde campholénique et, à notre connaissance, seuls deux documents dans l'art antérieur font référence à des alcools tertiaires dont les propriétés odorantes sont intéressantes [voir US 4,149,020 et US 4,174,287], ces alcools étant des dérivés campholéniques à chaîne ouverte.

Lorsqu'on analyse la littérature ayant trait à des composés de structure plus proche de celle du composé selon la présente invention, c'est-à-dire bicyclique, on se rend compte alors qu'il n'y a pas d'exemples d'alcools tertiaires olfactivement intéressants, malgré le nombre de composés divulgués. Dans ce contexte, il y a lieu de citer par exemple le brevet US 4,173,585 qui décrit, parmi d'autres, un mélange de composés de formule

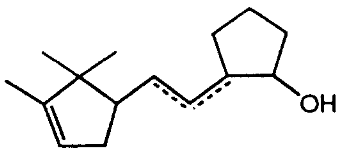

(Ia)

ayant une double liaison dans l'une des positions indiquées par les lignes pointillées, et un mélange de composés de formule

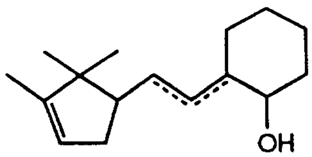

(Ib)

dans laquelle les lignes pointillées ont le sens indiqué ci-dessus. Ces deux mélanges possèdent, respectivement, une odeur boisée-santalée, avec un caractère "cyclamal/Lilial®", et une odeur de bois de cèdre, santalée douce.

Le brevet DE 29 35 683 décrit des composés de formule

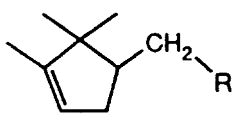

(II)

dans laquelle le symbole R représente un groupe cyclique de formule

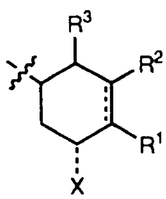

pouvant avoir une double liaison endocyclique dans la position indiquée par la ligne pointillée et dans laquelle la ligne pointillée exocyclique représente une liaison simple, X représentant OH, ou double, X représentant alors un atome d'oxygène, et les symboles $R^1$, $R^2$ et $R^3$ représentent un atome d'hydrogène ou un groupe alkyle inférieur, ou R représente un groupe cyclique de formule

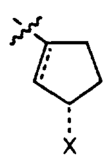

dans laquelle les lignes pointillées et le symbole X sont définis comme ci-dessus. Les composés de formule (II) possèdent des notes santalées de puissance et caractère variés.

Le 1,2-diméthyl-4-(2',2',3'-triméthyl-3'-cyclopentén-1'-yl)-2-cyclohexén-1-ol selon la présente invention appartient à un groupe de composés dont la structure moléculaire est plus rigide que celle des composés connus de formule (I) ou (II) cités, tout en maintenant la distance jugée optimale [voir Naipawer et al., référence citée] entre l'atome de carbone quaternaire 2' et le groupe fonctionnel 1. Il s'agit de composés

qui peuvent être représentés par le squelette de base suivant :

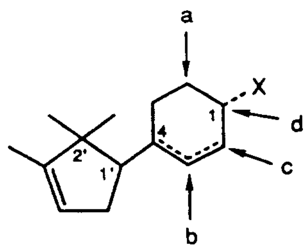

(III)

pouvant avoir une double liaison dans l'une des positions endocycliques indiquées par les lignes pointillées, la ligne pointillée exocyclique représentant une liaison simple lorsque X est un radical OH et une liaison double lorsque X représente un atome d'oxygène. Le cycle hexagonal peut posséder un ou plusieurs groupes méthyle substituants dans les positions a, b, c et d.

Au vu de l'art antérieur, on aurait pu s'attendre à ce que cette rigidification de la chaîne cyclique contenant le groupe fonctionnel produise un effet plus ou moins uniforme sur un groupe de composés ayant le même squelette de base (III) et qu'elle apporte, en réduisant le nombre de conformations possibles de ladite chaîne, une amélioration des qualités olfactives par rapport aux composés connus. Malgré cela, aucun composé odorant de structure (III) n'a été décrit dans l'art antérieur. Or, nous avons maintenant découvert que, contrairement à ce qu'on aurait pu prévoir, parmi nombre de composés de structure (III), dont quelques uns sont ici rapportés, seul le 1,2-diméthyl-4-(2',2',3'-triméthyl-3'-cyclopentén-1'-yl)-2-cyclohexén-1-ol possède des propriétés odorantes supérieures à celles des composés connus et une note odorante dont les caractères s'apparentent au mieux à ceux du bois de santal naturel. Comme il ressort du tableau présenté ci-après, d'autres composés de structure (III) présentent des notes de type santalé mais qui sont, soit trop faibles, soit moins intéressantes qualitativement.

## TABLEAU I

| Composés | Propriétés odorantes |
|---|---|
| 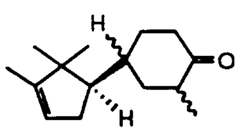 | cumin, animal, boisé, légérement santalé |
| 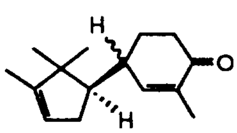 | sesquiterpènes, fruité, faible |
| 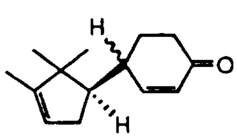 | boisé, fleuri, santalé, vague |
| 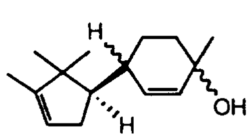 (cis) | boisé-faible, très vaguement santalé, fruité |

TABLEAU I (suite)

| Composés | Propriétés odorantes |
| --- | --- |
| 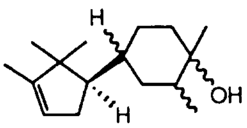 | boisé, phénolique, cuir, légèrement santalé |
| 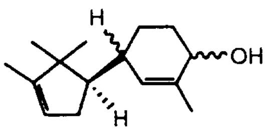 (cis : trans) (42 : 52) | légèrement santalé, faible |
| 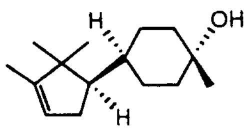 | faible |
| 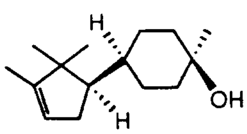 | faible, floral, sale |
| 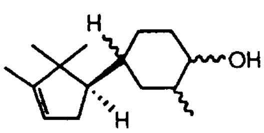 | fleuri, boisé, vaguement santalé, faible |
| 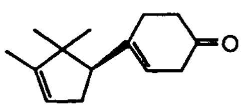 | iris, légèrement santalé |
| 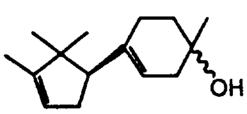 | faible, vaguement floral, très vaguement santalé |
| 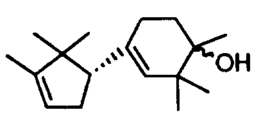 | boisé, vaguement santalé |

Au vu de ce tableau, les qualités olfactives du composé de l'invention sont encore plus surprenantes, puisque des composés de structure presque identique exhibent des caractères olfactifs peu distinctifs, trop faibles ou qui ne contiennent pas la note santalée recherchée.

Ceci a également été constaté lorsqu'on a comparé les propriétés odorantes du composé selon l'invention avec celles de composés connus, de structure proche, obéissant aux formules (Ib) et (II) citées auparavant. Par exemple, le (-)-(1'R)-2-[2-(2',2',3'-triméthyl-3'-cyclopentén-1'-yl)éthylidène]-1-cyclohexanol de formule

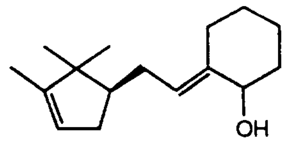

$[\alpha]^{20}_D = -3{,}4°$   c = 1,7%, dans le $CHCl_3$

que nous avons préparé comme il est décrit dans US 4,173,585, mais partant d'un produit de départ optiquement actif pur, possède une odeur faiblement boisée, résineuse, avec un très faible caractère santalé. De même, les (+)-(1'S)-3-(2',2',3'-triméthyl-3'-cyclopentén-1'-yl)méthyl-1-cyclohexanol et (+)-(1'R)-4-méthyl-3-(2',2',3'-triméthyl-3'-cyclopentén-1'-yl)méthyl-1-cyclohexanol de formules

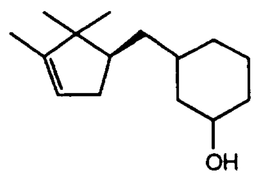

$[\alpha]^{20}_D = +9{,}3°$   c = 2,95%, dans le $CHCl_3$

et, respectivement

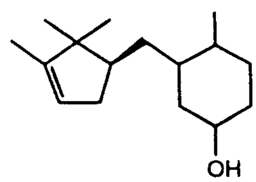

$[\alpha]^{20}_D = +0{,}2°$   c = 2,25%, dans le $CHCl_3$

obtenus selon DE 29 35 683 à partir de produits de départ optiquement actifs purs, se sont avérés posséder une odeur vaguement boisée-santalée, sans caractère et, respectivement, très vaguement santalée, encore plus faible que la précédente.

De par ses qualités olfactives, le 1,2-diméthyl-4-(2',2',3'-triméthyl-3'-cyclopentén-1'-yl)-2-cyclohexén-1-ol se prête particulièrement bien aux applications en parfumerie fine, pour la préparation de parfums et eaux de toilette de type varié, en particulier de type boisé-fleuri-oriental, compositions auxquelles il apporte un net caractère santalé, tout en exaltant l'aspect fleuri et animal de la composition.

La ténacité de sa note odorante le rend également très utile dans la parfumerie fonctionnelle, notamment pour le parfumage de détergents et adoucissants textiles. D'autres produits pouvant être avantageusement parfumés à l'aide du composé de l'invention incluent les savons, les gels de douche ou bain, les shampoings, les cosmétiques, les désodorisants corporels ou d'air ambiant ou les produits d'entretien.

Comme cela est souvent le cas en parfumerie, il peut être utilisé, en tant qu'ingrédient parfumant, soit seul, soit en mélange avec d'autres co-ingrédients parfumants, des solvants ou des adjuvants usuels.

Les concentrations dans lesquelles le composé de l'invention peut être utilisé pour les applications susmentionnées varient dans une gamme de valeurs très étendue, qui est fonction, parmi d'autres facteurs, de la nature du produit à parfumer et de l'effet parfumant désiré. A titre d'exemple, on peut citer des concentrations de l'ordre de 1 à 10% en poids, ou même plus, lorsqu'il est utilisé dans des bases et concentrés parfumants. Des valeurs nettement inférieures à celles citées peuvent être employées lors du parfumage d'articles divers tels que ceux mentionnés plus haut.

Il convient de noter que le composé selon l'invention peut se présenter sous différentes formes optiquement actives, en raison de la pluralité de centres chiraux dans la molécule. Comme il est décrit ci-dessous, ces différents isomères peuvent être préparés en utilisant les produits de départ sous des formes optiquement actives appropriées. Nous avons constaté que les isomères ainsi obtenus différaient tous les uns des autres d'un point de vue olfactif, ceci aussi bien en ce qui concernait le caractère de la note santalée que son intensité. D'une façon générale, cependant, il a été observé que ces différences étaient légères et que tous ces composés, ainsi que leurs mélanges, convenaient bien pour les applications en parfumerie selon l'invention. On a remarqué toutefois une préférence des parfumeurs envers les isomères de configuration (-)-(1'R) et, parmi ceux-ci, le (-)-(1'R)-cis-1,2-diméthyl-4-(2',2',3'-triméthyl-3'-cyclopentén-1'-yl)-2-cyclohexén-1-ol.

Lorsqu'il est fait mention dans la présente description du 1,2-diméthyl-4-(2',2',3'-triméthyl-3'-cyclopentén-1'-yl)-2-cyclohexén-1-ol, on veut faire référence à l'un quelconque de ses isomères optiquement actifs, ou à tout mélange de ceux-ci.

L'invention concerne également un procédé de préparation du 1,2-diméthyl-4-(2',2',3'-triméthyl-3'-cyclopentén-1'-yl)-2-cyclohexén-1-ol caractérisé en ce qu'on fait réagir la 2-méthyl-4-(2',2',3'-triméthyl-3'-cyclopentén-1'-yl)-2-cyclohexén-1-one avec un réactif de Grignard approprié, dans les conditions usuelles de ce type de réaction. La cyclohexénone de départ dans le procédé de l'invention peut se présenter sous différentes formes stéréoisomères optiquement actives, lesquelles conditionnent, bien évidemment, la configuration du produit final.

La 2-méthyl-4-(2',2',3'-triméthyl-3'-cyclopentén-1'-yl)-2-cyclohexén-1-one, sous la configuration désirée, ou sous forme d'un mélange de diastéréomères, peut être obtenue à partir de la forme stéréoisomère appropriée de l'aldéhyde campholénique, à l'aide de réactions de type conventionnel, telles qu'illustrées au schéma suivant. Dans ce schéma, la cyclohexénone susmentionnée est représentée, sous forme de l'isomère de configuration 1'R (mélange de diastéréomères 4R/4S), par le chiffre 6 et le composé selon l'invention, dans la configuration correspondante, est indiqué par le chiffre 22.

## Schéma

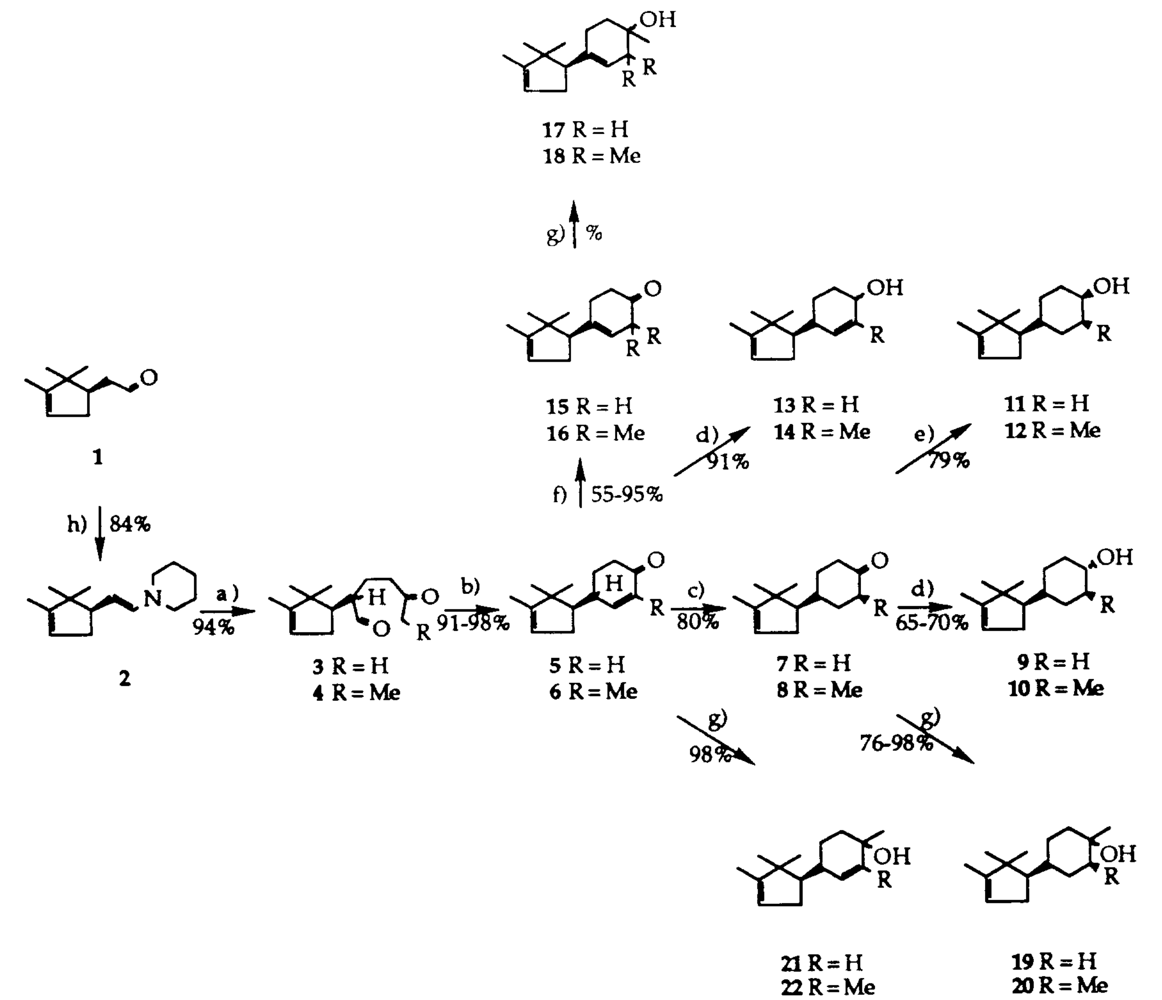


17 R = H
18 R = Me

15 R = H
16 R = Me

13 R = H
14 R = Me

11 R = H
12 R = Me

1

2

3 R = H
4 R = Me

5 R = H
6 R = Me

7 R = H
8 R = Me

9 R = H
10 R = Me

21 R = H
22 R = Me

19 R = H
20 R = Me

a) 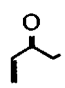 , cyclohexane, AcOH

b) pTsOH, cyclohexane

c) $H_2$/Raney Ni, EtOH

d) $LiAlH_4$, $Et_2O$

e) L-Sélectride, THF -70°

f) DMSO, KOtBu, RI

g) MeMgI, éther

h) pipéridine, $H^{\oplus}$, toluène

Ce schéma montre également les réactions de type conventionnel qui permettent de préparer, à partir de la même cyclohexénone de départ, de nombreux composés obéissant à la structure de base (III), notamment ceux cités au Tableau I.

Le procédé selon l'invention sera maintenant décrit de façon plus détaillée à l'aide des exemples présentés ci-après, dans lesquels les températures sont indiquées en degrés centigrades et les abréviations ont le sens usuel dans l'art.

D'autres exemples présentés plus loin illustrent les applications en parfumerie du composé faisant l'objet de la présente invention.

Exemple 1

Préparation de (-)-(1'R)-cis-1,2-diméthyl-4-(2',2',3'-triméthyl-3'-cyclopentén-1'-yl)-2-cyclohexén-1-ol et de (-)-(1'R)-trans-1,2-diméthyl-4-(2',2',3'-triméthyl-3'-cyclopentén-1'-yl)-2-cyclohexén-1-ol

a) Préparation de (+)-(1'R,E)-N-[2-(2',2',3'-triméthyl-3'-cyclopentén-1'-yl)éthényl] pipéridine

Dans un ballon de 3 l, on a placé 608 g (4 mole) d'aldéhyde campholénique (+), 2 l de toluène, 408 g (4,8 mole) de pipéridine et 3 g d'acide p-toluène sulfonique. On a chauffé à reflux durant 3h avec un séparateur d'eau. On a ajouté à température ambiante 3 g de $Na_2CO_3$ puis, après 15 minutes, filtré et évaporé pour obtenir 921 g d'une huile qui a été distillée pour obtenir 733,87 g de la pipéridine désirée.

P. éb. 136-138°/4 Pa; rend. : 84%

$[\alpha]^{20}_D$ = +13,6° c = 2,22%, dans le $CHCl_3$

IR : 2940, 1640, 1450, 1380, 1190 $cm^{-1}$

RMN($^1$H, 360MHz) : 0,75(s, 3H) ; 0,92(s, 3H) ; 1,61(d, J=2Hz, 3H) ; 2,77(t, J=7Hz, 4H) ; 4,42(dxd, $J_1$=15Hz, $J_2$=8Hz, 1H) ; 5,24(s, 1H); 5,82(d, J=15Hz, 1H) $\delta$ ppm

SM : 219($M^+$, 64), 204(100), 176(20), 134(44), 122(85), 111(68), 96(60), 91(50), 84(56), 79(55), 41(68).

b) Préparation de (+)-(1'R)-5-oxo-2-(2',2',3'-triméthyl-3'-cyclopentén-1'-yl)heptanal

Dans un ballon de 250 ml, on a placé, sous $N_2$, 36,5 g (0,167 mole) de la pipéridine préparée selon a) dans 70 ml de cyclohexane. Sous agitation, on a ajouté 16,9 g (0,2 mole) d'éthyle vinylcétone et porté à reflux durant 24 heures. On a ajouté à température ambiante 25 ml d'acide acétique 50%, puis porté à reflux durant 15 minutes. Après avoir séparé les phases, on a lavé la phase organique avec 3 x HCl 15%, 2 x avec de l'eau, 2 x avec NaCl saturé pour obtenir, après séchage ($Na_2SO_4$) et évaporation, 39,28 g de produit. Après distillation, on a obtenu 36,92 g de l'heptanal désiré sous forme d'un mélange de diastéréoisomères 4 : 3. Ce mélange de diastéréoisomères n'est pas séparable par des techniques de séparation simples et a été utilisé tel quel dans la poursuite de la synthèse.

P. éb. 103-118°/4 Pa; rend. : 94%

$[\alpha]^{20}_D$ = +5,86° c = 3,68%, dans le $CHCl_3$

IR : 2950, 1720, 1460, 1360, 1120, 1020 $cm^{-1}$

RMN($^1$H, 360MHz) : isomère majoritaire : 0,83(s, 3H); 0,96(s, 3H) ; 1,04(t, J=7Hz, 3H) ; 1,59(s, 3H) ; 5,24(s, 1H) ; 9,52(d, J=4Hz, 1H) $\delta$ ppm isomère minoritaire : 0,94(s, 3H) ; 1,05(t, J=7Hz, 3H) ; 1,11(s, 3H) ; 1,58(s, 3H) ; 5,21(s, 1H); 9,55(d, J=4Hz, 1H) $\delta$ ppm

RMN($^{13}$C) : isomère majoritaire : 7,77(q) ; 12,49(q) ; 20,15(q) ; 21,79(t) ; 22,89(q) ; 33,77(t) ; 36,11(t) ; 39,31(t) ; 46,99(s) ; 50,13(d) ; 53,8(d) ; 121,12(d) ; 148,55(s) ; 205,48(d) ; 210,39(s) $\delta$ ppm

isomère minoritaire : 7,77(q) ; 12,4(q) ; 20,22(q) ; 22,58(t) ; 27,16(q) ; 32,93(t) ; 36,4(t) ; 39,55(t) ; 47,7(s) ; 51,1(d) ; 52,78(d) ; 121,45(d) ; 148,5(s) ; 204,7(d) ; 210,47(s) $\delta$ ppm

SM : 236($M^+$, 0), 121(12), 108(100), 93(65), 57(22).

Odeur : sesquiterpènes, faible.

c) Préparation de (-)-(1'R)-2-méthyl-4-(2',2',3'-triméthyl-3'-cyclopentén-1'-yl)-2-cyclohexén-1-one

On a chauffé à reflux un mélange de 26 g (0,11 mole) de l'heptanal préparé selon b), 1 g d'acide p-toluène sulfonique et 50 ml de cyclohexane dans un ballon de 100 ml équipé d'un séparateur d'eau durant 4 heures.

On a lavé 2 x avec $NaHCO_3$, 2 x avec $H_2O$ et 2 x avec NaCl saturé. Après séchage sur $Na_2SO_4$, on a évaporé pour obtenir 25,26 g de produit qui ont été distillés pour fournir 21,79 g de la cyclohexénone désirée sous forme d'un mélange de diastéréoisomères 1,1 : 1.

P. éb. 130°/13Pa ; rend. : 91%

$[\alpha]^{20}_D$ = -14,6° c = 3,63%, dans le $CHCl_3$

IR : 3020, 2950, 1695, 1450, 1360, 900, 800 $cm^{-1}$

RMN($^1$H, 360MHz) : diastéréomère majoritaire : 0,98(s, 3H) ; 1,12(s, 3H) ; 1,61(s, 3H) ; 1,80(s, 3H) ; 5,24-(s, 1H) ; 6,9(s, 1H) $\delta$ ppm

diastéréomère minoritaire : 0,96(s, 3H) ; 1,1(s, 3H) ; 1,61(s, 3H) ; 1,77(s, 3H) ; 5,24(s, 1H) ; 6,73(s, 1H) $\delta$ ppm

RMN($^{13}$C) : diastéréomère majoritaire : 12,5(q) ; 19,18(q) ; 20,03(q) ; 27,64(q) ; 28,35(t) ; 34,75(t) ; 36,44-(t) ; 37,75(d) ; 46,92(s) ; 54(d) ; 121,2(d) ; 134,7(s) ; 148,55(s) ; 149,84(d) ; 199,75(s) $\delta$ ppm

diastéréomère minoritaire : 12,5(q) ; 16,18(q) ; 19,78(q) ; 27,35(q) ; 29,53(t) ; 34,18(t) ; 37,04(t) ; 38,09(d) ; 47(s) ; 53,09(d) ; 121,35(d) ; 135,3(s) ; 148,5(s) ; 149,62(d) ; 199,75(s) $\delta$ ppm

SM : 218($M^+$, 5), 203(8), 189(21), 109(100), 95(50), 81(62), 67(77).

Odeur : sesquiterpènes, fruitée, faible.

d) Dans un ballon de 250 ml sous $N_2$ on a placé 0,96g (0,04 mole) de Mg et 50 ml d'éther, ajouté goutte à goutte 5,68 g (0,04 mole) de $CH_3I$ puis, après disparition du Mg, additionné une solution de 6,5g (0,03 mole) de la cyclohexénone préparée sous c) dans 20 ml d'éther en 20 minutes et porté à reflux durant une heure. On a ajouté 30 ml d'une solution saturée au $NH_4Cl$, extrait avec 4 x 10 ml d'éther, lavé avec de l'eau puis une solution saturée de NaCl, séché sur $Na_2SO_4$ et évaporé pour obtenir 7,43 g d'un mélange d'isomères cis : trans 1 : 2 du (1'R)-1,2-diméthyl-4-(2',2',3'-triméthyl-3'-cyclopentén-1'-yl)-2-cyclohexén-1-ol. Ce mélange a été soumis à une chromatographie sur colonne de $SiO_2$ en utilisant comme éluant un mélange toluène/acétate d'éthyle 8 : 2 pour fournir 2,1 g de l'isomère cis, sous forme d'un mélange 1,6 : 1 de diastéréomères, et 3,8 g de l'isomère trans, sous forme d'un mélange 1,1 : 1 de diastéréomères.

P. éb. 160-180°/13 Pa ; rend. : 98%

(-)-(1'R)-cis-1,2-diméthyl-4-(2',2',3'-triméthyl-3'-cyclopentén-1'-yl)-2-cyclohexén-1-ol

$[\alpha]^{20}_D$ = -12,3° c = 3,05%, dans le $CHCl_3$

IR : 3400, 2940, 1440, 1360, 940, 915, 805 $cm^{-1}$

RMN($^1$H, 360MHz) : diastéréomère majoritaire : 0,92(s, 3H) ; 1,13(s, 3H) ; 1,29(s, 3H) ; 1,59(s, 3H) ; 1,77-(s, 3H) ; 5,22(s, 1H) ; 5,64(s, 1H) δ ppm

diastéréomère minoritaire : 0,90(s, 3H); 1,08(s, 3H); 1,29(s, 3H); 1,59(s, 3H) ; 1,77(s, 3H) ; 5,22(s, 1H) ; 5,45(s, 1H) δ ppm

RMN($^{13}$C) : diastéréomère majoritaire : 12,5(q) ; 18,19(q) ; 19,86(q) ; 25,04(t) ; 27,13(q) ; 27,91(q) ; 34,62-(t) ; 38,06(d) ; 38,48(t) ; 46,98(s) ; 54,57(d) ; 69,65(s) ; 121,38(d) ; 129,77(d) ; 137,52(s) ; 148,67(s) δ ppm

diastéréomère minoritaire : 12,5(q) ; 18,19(q) ; 19,58(q) ; 26,13(t) ; 27,37(q) ; 27,91(q) ; 34,34(t) ; 38,11(d) ; 38,63(t) ; 47,22(s) ; 54,27(d) ; 69,44(s) ; 121,48(d) ; 129,47(d) ; 137,86(s) ; 148,67(s) δ ppm

SM : 220($M^+$, 0), 216(8), 108(100), 91(53), 67(48), 43(35).

(-)-(1'R)-trans-1,2-diméthyl-4-(2',2',3'-triméthyl-3'-cyclopentén-1'-yl)-2-cyclohexén-1-ol

$[\alpha]^{20}_D$ = -12,6° c = 3,2%, dans le $CHCl_3$

IR : 3400, 2950, 1440, 1360, 1110 $cm^{-1}$

RMN($^1$H, 360MHz) : diastéréomère majoritaire : 0,92(s, 3H) ; 1,09(s, 3H) ; 1,3(s, 3H) ; 1,59(s, 3H) ; 1,76(s, 3H) ; 5,22(s, 1H) ; 5,59(s, 1H) δ ppm

diastéréomère minoritaire : 0,89(s, 3H) ; 1,06(s, 3H) ; 1,29(s, 3H) ; 1,59(s, 3H) ; 1,64(s, 3H) ; 5,22(s, 1H) ; 5,38(s, 1H) δ ppm

RMN($^{13}$C) : diastéréomère majoritaire : 12,5(q) ; 17,68(q) ; 19,75(q) ; 26,0(t) ; 26,8(q) ; 27,34(q) ; 34,31(t) ; 37,51(d) ; 38,86(t) ; 47,22(s) ; 54,2(d) ; 70,92(s) ; 121,55(d) ; 128,19(d) ; 138,61(s) ; 148,52(s) δ ppm

diastéréomère minoritaire : 12,5(q) ; 17,68(q) ; 19,61(q) ; 26,86(q) ; 27,49(t) ; 27,64(q) ; 34,57(t) ; 37,41(d) ; 38,35(t) ; 46,88(s) ; 54,49(d) ; 71,17(s) ; 121,25(d) ; 128,67(d) ; 138,02(s) ; 148,85(s) δ ppm

SM : diastéréomère minoritaire : 234($M^+$, 3), 216(13), 109(100), 91(57), 67(65), 43(80).

diastéréomère majoritaire : 234($M^+$, 8), 216(9), 201(10), 109(100), 91(50), 67(54), 43(72).

## Exemple 2

### Préparation de (+)-(1'S)-cis-1,2-diméthyl-4-(2',2',3'-triméthyl-3'-cyclopentén-1'-yl)-2-cyclohexén-1-ol et de (+)-(1'S)-trans-1,2-diméthyl-4-(2',2',3'-triméthyl-3'-cyclopentén-1'-yl)-2-cyclohexén-1-ol

Ces composés ont été préparés de façon identique à celle décrite dans l'exemple 1, mais en utilisant comme produit de départ l'aldéhyde campholénique (-) pour la préparation de la (-)-(1'S,E)-N-[2-(2',2',3'-triméthyl-3'-cyclopentén-1'-yl)éthényl]pipéridine. Les données analytiques des intermédiaires et produits finaux obtenus étaient identiques à celles de leurs énantiomères correspondants décrits dans l'exemple 1, à l'exception des angles de rotation optique, qui étaient les suivants :

a) (-)-(1'S,E)-N-[2-(2',2',3'-triméthyl-3'-cyclopentén-1'-yl)éthényl]pipéridine

$[\alpha]^{20}_D$ = -18,77° (à l'état pur)

b) (-)-(1'S)-5-oxo-2-(2',2',3'-triméthyl-3'-cyclopentén-1'-yl)heptanal (mélange de diastéréomères)

$[\alpha]^{20}_D$ = -6,23° c = 3,75%, dans le $CHCl_3$

c) (+)-(1'S)-2-méthyl-4-(2',2',3'-triméthyl-3'-cyclopentén-1'-yl)-2-cyclohexén-1-one (mélange de diastéréomères)

$[\alpha]^{20}_D$ = +20,4° (à l'état pur)

d) (+)-(1'S)-cis-1,2-diméthyl-4-(2',2',3'-triméthyl-3'-cyclopentén-1'-yl)-2 cyclohexén-1-ol (mélange de diastéréomères)

$[\alpha]^{20}_D$ = +15,3° c = 1,67%, dans le $CHCl_3$

e) (+)-(1'S)-trans-1,2-diméthyl-4-(2',2',3'-triméthyl-3'-cyclopentén-1'-yl)-2-cyclohexén-1-ol (mélange de diastéréomères)
$[\alpha]^{20}_D$ = +28,95° c = 1,18%, dans le $CHCl_3$

Exemple 3

Préparation d'une composition parfumante

On a préparé une composition de base pour une eau de toilette féminine en mélangeant les ingrédients suivants :

| Ingrédients | Parties en poids |
|---|---|
| Acétate de benzyle | 80 |
| Acétate de géranyle | 20 |
| Acétate de styrallyle | 20 |
| Aldhéhyde hexylcinnamique | 100 |
| Glycolate d'allyl amyle à 10%* | 150 |
| γ-Undécalactone à 10%* | 100 |
| Essence de bergamote | 500 |
| Citronellol | 50 |
| Coumarine | 150 |
| Exaltolide ® 1) | 200 |
| Isoeugénol | 25 |
| Absolue de Jasmin | 75 |
| Iralia ® 2) | 600 |
| Muscone | 100 |
| Hédione ® 3) | 1220 |
| Eugénol | 50 |
| Salicylate de benzyle | 100 |
| Salicylate de cis-3-hexénol | 60 |
| Essence de santal | 900 |
| Vanilline | 100 |
| Essence d'Ylang | 150 |
| Essence de citron de Sicile | 50 |
| Total | 4800 |

* dans le dipropylèneglycol (DIPG)
1) cyclopentadécanolide ; origine : Firmenich SA, Genève, Suisse
2) méthylionone (mélange isomérique) ; origine : Firmenich SA, Genève, Suisse
3) dihydrojasmonate de méthyle ; origine : Firmenich SA, Genève, Suisse

A cette composition de base de type fleuri-oriental-santalé, on a ajouté 200 parties en poids de 1,2-diméthyl-4-(2',2',3'-triméthyl-3'-cyclopentén-1'-yl)-2-cyclohexén-1-ol. On a ainsi obtenu une composition nouvelle dont la note odorante était devenue nettement plus santalée, plus arrondie et plus montante. En plus, les notes florales et musquées de la composition de base se sont trouvées exaltées dans la nouvelle composition, en résultat de l'adjonction du composé susmentionné.
Cet effet de renforcement du caractère santalé de la composition par adjonction du composé de l'invention est très net. Ainsi, il a été constaté que, si au lieu d'ajouter ce dernier, on double la quantité d'essence de santal dans la composition de base, la note de cette composition devient plate, doucereuse, sans caractère. On n'observe pas non plus, l'exaltation du caractère boisé-floral de la composition, si remarqué lors de l'addition du 1,2-diméthyl-4-(2',2',3'-triméthyl-3'-cyclopentén-1'-yl)-2-cyclohexén-1-ol à la composition de base.

Exemple 4

Préparation d'une composition parfumante

On a préparé une composition parfumante de base, destinée à un adoucissant textile, en mélangeant les ingrédients suivants :

| Ingrédients | Parties en poids |
|---|---|
| Acétate de benzyle | 5 |
| Acétate de carbinol | 3 |
| Aldéhyde anisique redistillé à 10%* | 5 |
| Aldéhyde hexylcinnamique | 5 |
| Benzoate de méthyle à 10%* | 3 |
| Citronellol | 5 |
| Acétate de verdyle | 3 |
| Acétate de p-tert-butylcyclohexyle | 7 |
| Ethylvanilline à 1%* | 3 |
| Galaxolide ® [1] 50 | 7 |
| Géraniol | 5 |
| Isoraldéine ® [2] 70 | 3 |
| Lilial ® [3] | 5 |
| Linalol | 4 |
| Lyral ® [4] | 2 |
| Méthylnaphtylcétone cryst. | 1 |
| Carbonate de méthyl octyne à 1%* | 4 |
| Phénéthylol | 10 |
| Rosinol | 3 |
| Salicylate de benzyle | 5 |
| Cedroxyde ® [5] | 5 |
| Salicylate d'amyle | 6 |
| Total | 99 |

* dans le dipropylèneglycol (DIPG)
1) 1,3,4,6,7,8-hexahydro-4,6,6,7,8,8-hexaméthyl-cyclopenta[g]isochromène; origine : IFF Inc., USA
2) iso-méthylionone ; origine : L. Givaudan, Vernier, Suisse
3) 3-(4-tert-butyl-1-phényl)-2-méthylpropanal ; origine : L. Givaudan, Vernier, Suisse
4) 4-(4-hydroxy-4-méthylpentyl)-3-cyclohexène-1-carboxaldéhyde ; origine : IFF Inc., USA
5) triméthyl cyclododécatriène époxyde ; origine : Firmenich SA, Genève, Suisse

A cette composition de base de type floral-rosé, musqué, on a ajouté une partie en poids de 1,2-diméthyl-4-(2',2',3'-triméthyl-3'-cyclopentén-1'-yl)-2-cyclohexén-1-ol. On a ainsi obtenu une composition nouvelle dont la note olfactive, bien que n'ayant pas changé radicalement par rapport à celle de la composition de base, était devenue plus chaude, avec une sous-note boisée nettement perceptible. On a constaté que cet effet odorant était encore plus perceptible sur du linge humide, fraîchement sorti de la machine à laver et qu'il était en plus très durable sur le linge sec, démontrant l'excellente substantivité de la note odorante impartie par le composé de l'invention susmentionné.

**Revendications**

**1.** 1,2-Diméthyl-4-(2',2',3'-triméthyl-3'-cyclopentén-1'-yl)-2-cyclohexén-1-ol.

**2.** Le composé selon la revendication 1, sous forme de l'un de ses isomères optiquement actifs suivants :
a) (-)-(1'R)-cis-1,2-diméthyl-4-(2',2',3'-triméthyl-3'-cyclopentén-1'-yl)-2-cyclohexén-1-ol;

b) (-)-(1'R)-trans-1,2-diméthyl-4-(2',2',3'-triméthyl-3'-cyclopentén-1'-yl)-2-cyclohexén-1-ol ;
c) (+)-(1'S)-cis-1,2-diméthyl-4-(2',2',3'-triméthyl-3'-cyclopentén-1'-yl)-2-cyclohexén-1-ol;
d) (+)-(1'S)-trans-1,2-diméthyl-4-(2',2',3'-triméthyl-3'-cyclopentén-1'-yl)-2-cyclohexén-1-ol;
ou d'un mélange quelconque de deux ou plusieurs de ces isomères.

**3.** Utilisation du composé selon la revendication 1 ou 2 à titre d'ingrédient parfumant.

**4.** Composition parfumante ou article parfumant contenant à titre d'ingrédient parfumant le composé selon la revendication 1 ou 2.

**5.** A titre d'article parfumé selon la revendication 4, un parfum ou une eau de toilette, un gel de douche ou bain, un shampoing, une préparation cosmétique, un désodorisant corporel ou d'air ambiant, un détergent ou un adoucissant textile, un produit d'entretien.

**6.** Procédé de préparation du 1,2-diméthyl-4-(2',2',3'-triméthyl-3'-cyclopentén-1'-yl)-2-cyclohexén-1-ol, caractérisé en ce qu'on fait réagir la 2-méthyl-4-(2',2',3'-triméthyl-3'-cyclopentén-1'-yl)-2-cyclohexén-1-one avec un réactif de Grignard approprié, dans les conditions usuelles de ce type de réaction.

**7.** Procédé selon la revendication 6, caractérisé en ce que la 2-méthyl-4-(2',2',3'-triméthyl-3'-cyclopentén-1'-yl)-2-cyclohexén-1-one est utilisée sous forme de l'un de ses isomères optiquement actifs ou d'un mélange de deux ou plusieurs de ces isomères.

**8.** 2-Méthyl-4-(2',2',3'-triméthyl-3'-cyclopentén-1'-yl)-2-cyclohexén-1-one.

## Claims

**1.** 1,2-Dimethyl-4-(2',2',3'-trimethyl-3'-cyclopenten-1'-yl)-2-cyclohexen-1-ol.

**2.** The compound according to claim 1, in the form of one of the following optically active isomers:
a) (-)-(1'R)-cis-1,2-dimethyl-4-(2',2',3'-trimethyl-3'-cyclopenten-1'-yl)-2-cyclohexen-1-ol;
b) (-)-(1'R)-trans-1,2-dimethyl-4-(2',2',3'-trimethyl-3'-cyclopenten-1'-yl)-2-cyclohexen-1-ol;
c) (+)-(1'S)-cis-1,2-dimethyl-4-(2',2',3'-trimethyl-3'-cyclopenten-1'-yl)-2-cyclohexen-1-ol, and
d) (+)-(1'S)-trans-1,2-dimethyl-4-(2',2',3'-trimethyl-3'-cyclopenten-1'-yl)-2-cyclohexen-1-ol;
or of any mixture of two or more of these isomers.

**3.** Use of a compound according to claim 1 or 2 as a perfuming ingredient.

**4.** A perfuming composition or a perfumed article containing as a perfuming ingredient the compound according to claim 1 or 2.

**5.** As perfumed article according to claim 4, a perfume or a cologne, a shower or bath gel, a shampoo, a cosmetic preparation, an air or body deodorant, a detergent or a fabric softener, or a household product.

**6.** A process for the preparation of 1,2-dimethyl-4-(2',2',3'-trimethyl-3'-cyclopenten-1'-yl)-2-cyclohexen-1-ol, characterized in that 2-methyl-4-(2',2',3'-trimethyl-3'-cyclopenten-1-yl)-2-cyclohexen-1-one is reacted with an appropriate Grignard reagent, under the usual conditions of this type of reaction.

**7.** A process according to claim 6, characterized in that 2-methyl-4-(2',2',3'-trimethyl-3'-cyclopenten-1'-yl)-2-cyclohexen-1-one is used in the form of any one of its optically active isomers or of a mixture of two or more of said isomers.

**8.** 2-Methyl-4-(2',2',3'-trimethyl-3'-cyclopenten-1'-yl)-2-cyclohexen-1-one.

## Patentansprüche

**1.** 1,2-Dimethyl-4-(2',2',3'-trimethyl-3'-cyclopenten-1'-yl)-2-cyclohexen-1-ol.

**2.** Die Verbindung gemäß Anspruch 1, in Form eines der folgenden optisch aktiven Isomeren:
a) (-)-(1'R)-cis-1,2-Dimethyl-4-(2',2',3'-trimethyl-3'-cyclopenten-1'-yl)-2-cyclohexen-1-ol;
b) (-)-(1'R)-trans-1,2-Dimethyl-4-(2',2',3'-trimethyl-3'-cyclopenten-1'-yl)-2-cyclohexen-1-ol;
c) (+)-(1'S)-cis-1,2-Dimethyl-4-(2',2',3'-trimethyl-3'-cyclopenten-1'-yl)-2-cyclohexen-1-ol, und
d) (+)-(1'S)-trans-1,2-Dimethyl-4-(2',2',3'-trimethyl-3'-cyclopenten-1'-yl)-2-cyclohexen-1-ol;
oder in Form eines Gemisches von zwei oder mehreren dieser Isomeren.

**3.** Verwendung einer Verbindung gemäß Anspruch 1 oder 2, als Riechstoffbestandteil.

**4.** Riechstoffkomposition oder parfümierter Artikel enthaltend als Riechstoffbestandteil die Verbindung gemaß Anspruch 1 oder 2.

**5.** Als parfümierter Artikel gemäß Anspruch 4, ein Parfum oder ein Toilettwasser, eine Bade- oder Duschgel, ein Schampoo, ein kosmetisches Präparat, ein Desodorierungsmittel für den Körper oder die Raumluft, ein Reinigungsmittel oder ein Textilauffrischungsmittel, ein Pflegeprodukt.

**6.** Verfahren zur Herstellung von 1,2-Dimethyl-4-(2',2',3'-trimethyl-3'-cyclopenten-1'-yl)-2-cyclohexen-1-ol, dadurch gekennzeichnet, daß man 2-Methyl-4-(2',2',3'-trimethyl-3'-cyclopenten-1'-yl)-2-cyclohexen-1-on mit einem geeigneten Grignardsreagens, unter der üblichen Bedingungen dieser Reaktion, reagiert.

**7.** Verfahren gemäß Anspruch 6, dadurch gekennzeichnet, daß man 2-Methyl-4-(2',2',3'-trimethyl-3'-cyclopenten-1'-yl)-2-cyclohexen-1-on in Form eines seiner optisch aktiven Isomeren oder in Form eines Gemisches von zwei oder mehreren dieser Isomeren verwendet.

**8.** 2-Methyl-4-(2',2',3'-trimethyl-3'-cyclopenten-1'-yl)-2-cyclohexen-1-on.